# EUROPEAN PATENT APPLICATION

(11) **EP 2 055 704 A1**
(43) Date of publication of application: **06.05.2009**
(21) Application number: 07792594.9
(22) Date of filing: 16.08.2007
(51) Int. Cl.: C07D 401/14, C07D 417/14, C09K 11/06, H01L 51/50

(54) **COMPOUND HAVING TRIAZINE RING STRUCTURE SUBSTITUTED WITH PYRIDYL GROUP AND ORGANIC ELECTROLUMINESCENT DEVICE**

(30) Priority: 21.08.2006 JP 2006223725
(71) Applicant: Hodogaya Chemical Co., Ltd., Tokyo 105-0011 (JP); National University Corporation Shinshu University, Matsumoto-shi, Nagano 3908621 (JP)
(72) Inventor: YOKOYAMA, Norimasa, Ibaraki 305-0841 (JP); AKISADA, Toru, Yamaguchi (JP); NAGAOKA, Makoto, Ibakari 305-0841 (JP); TANIGUCHI, Yoshio, Ueda-city, Nagano (JP); ICHIKAWA, Musubu, Ueda-city, Nagano (JP)
(74) Representative: Müller-Boré & Partner Patentanwälte
(86) International application number: PCT/JP2007/065964
(87) International publication number: WO 2008/023628

(57) **Abstract**

The present invention is to provide an organic compound having excellent characteristics as a material for an organic EL device having a high efficiency and a high durability, and to provide an organic EL device having a high efficiency and a high durability using the compound. The invention relates to a compound having a triazine ring structure having pyridyl groups attached thereto, which is represented by the general formula (1); and to an organic electroluminescent device comprising a pair of electrodes and at least one organic layer interposed between the electrodes, wherein the compound is used as a constituent material of at least one of the organic layer(s): 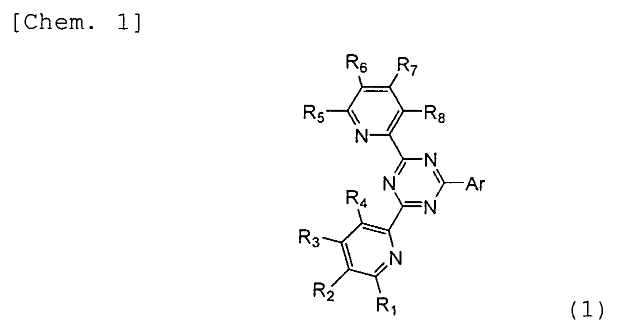 wherein Ar represents a substituted or unsubstituted aromatic hydrocarbon group, a substituted or unsubstituted aromatic heterocyclic group, or a substituted or unsubstituted condensed polycyclic aromatic group; R1 to R8 may be the same or different from one another and each independently represents a hydrogen atom, a fluorine atom, a trifluoromethyl group, a cyano group, an alkyl group, a substituted or unsubstituted aromatic hydrocarbon group, a substituted or unsubstituted aromatic heterocyclic group, or a substituted or unsubstituted condensed polycyclic aromatic group.

## Description

### TECHNICAL FIELD

The present invention relates to a compound suitable for an organic electroluminescent (EL) device which is a self-luminescent device suitable for various displaying devices and to a device. More specifically, it relates to a compound having a triazine ring structure having substituted or unsubstituted pyridyl groups attached thereto, and to an organic EL device using the compound.

### BACKGROUND ART

Since organic EL devices are self-luminescent devices, they are bright and excellent in visibility as compared with liquid-crystalline devices and capable of giving clear display, so that the organic EL devices have been actively studied.

In 1987, C. W. Tang et al. of Eastman Kodak Company put an organic EL device using organic materials into practical use by developing a device having a multilayered structure wherein various roles are assigned to respective materials. They formed a lamination of a fluorescent material capable of transporting electrons and an organic material capable of transporting holes, so that both charges are injected into the layer of the fluorescent material to emit light, thereby achieving a high luminance of 1000 cd/m² or more at a voltage of 10 V or lower (see, e.g., Patent Documents 1 and 2).

Patent Document 1: JP-A-8-48656
Patent Document 2: Japanese Patent No. 3194657

To date, many improvements have been performed for practical utilization of the organic EL devices, and high efficiency and durability have been achieved by an electroluminescent device wherein an anode, a hole-injecting layer, a hole-transporting layer, an emitting layer, an electron-transporting layer, an electron-injecting layer, and a cathode are sequentially provided on a substrate, to further segmentalize various roles (see, e.g., Non-Patent Document 1).

Non-Patent Document 1: Japan Society of Applied Physics Ninth Workshop Preprint, pp. 55-61 (2001)

Further, for the purpose of further improvement of luminous efficiency, utilization of triplet exciton has been attempted and utilization of a phosphorescent material has been investigated (see, e.g., Non-Patent Document 2).

Non-Patent Document 2: Japan Society of Applied Physics Ninth Workshop Preprint, pp. 23-31 (2001)

The emitting layer can be also prepared by doping a carrier-transporting compound, generally called a host material, with a fluorescent material or a phosphorescent material. As described in the above-mentioned Workshop Preprints, the choice of the organic materials in organic EL devices remarkably affects efficiency and durability of the devices.

In the organic EL devices, the charges injected from the both electrode are recombined in the emitting layer to attain emission. However, since the mobility of holes is higher than the mobility of electrons, a problem of reduction in efficiency caused by a part of holes passing through the emitting layer arises. Therefore, it is required to develop an electron-transporting material in which the mobility of electrons is high.

A representative emitting material, tris(8-hydroxyquinoline)aluminum (hereinafter referred to as Alq) is commonly used also as an electron-transporting material but it cannot be considered that the material has hole-blocking capability.

As a measure to prevent the passing of a part of holes through the emitting layer and to improve probability of charge recombination in the emitting layer, there is a method of inserting a hole-blocking layer. As hole-blocking materials, there have been hitherto proposed triazole derivatives (see, e.g., Patent Document 3), bathocuproine (hereinafter, referred to as BCP), a mixed ligand complex of aluminum (BAlq) (see, e.g., Non-Patent Document 2), and the like.

Patent Document 3: Japanese Patent No. 2734341

On the other hand, compounds having a triazine ring have been proposed as electron-transporting materials owing to the electron-withdrawing property of the triazine ring, but they are insufficient as the electron-transporting materials because of exciplex formation and formation of charge transfer complexes with neighboring organic layers (see, e.g., Non-Patent Documents 3 and 4) and thus they have been proposed to use as host materials of phosphorescence devices (see, e.g., Non-Patent Document 5).

Non-Patent Document 3: J. M. Lupton et al., J. Mater. Chem. 10, 876 (2000)
Non-Patent Document 4: J. Pang et al., J. Mater. Chem. 12, 206 (2002)
Non-Patent Document 5: Chihaya Adachi et al., J. Mater. Chem. 16, 1285 (2004)

Furthermore, ditriazine derivatives have been proposed as electron-transporting materials having higher mobility than Alq (see, e.g., Non-Patent Document 6), but are not sufficient as compared with the mobility of holes possessed by common hole-transporting materials such as NPD and TPD. Further, all the materials are deficient in film stability or are insufficient in function of blocking holes.

Non-Patent Document 6: The Chemical Society of Japan, 86th Spring Meeting Preprint, p. 530 (2006)

At present, a commonly used hole-blocking material is BCP but, since it is not sufficiently stable material, it cannot be considered that it sufficiently functions as a hole-blocking layer and thus satisfactory device properties have not been obtained.

In order to improve device properties of the organic EL devices, it is desired to develop an organic compound which is excellent in electron-injection/transport performances and hole-blocking ability and is highly stable in a thin-film state.

### DISCLOSURE OF THE INVENTION

### PROBLEMS TO BE SOLVED BY THE INVENTION

Objects of the invention are to provide an organic compound which has excellent characteristics, i.e., is excellent in electron-injection/transport performances, has hole-blocking ability and is highly stable in a thin-film state, as a material for an organic EL device having a high efficiency and a high durability, and also to provide an organic EL device having a high efficiency and a high durability using the compound. As physical properties of the organic compound suitable for the invention, there may be mentioned (1) good electron injection characteristic, (2) high electron mobility, (3) excellent hole-blocking ability, (4) good stability in a thin-film state, and (5) excellent thermal resistance.
In addition, as physical properties of the device suitable for the invention, there may be mentioned (1) high luminous efficiency, (2) low emission initiation voltage, (3) low practical driving voltage, and (4) high maximum emission luminance.

### MEANS FOR SOLVING THE PROBLEMS

Thus, in order to achieve the above objects, the present inventors have designed and chemically synthesized compounds having a triazine ring structure having substituted or unsubstituted pyridyl groups attached thereto, with focusing on the fact that the nitrogen atom of the pyridine ring which exhibits affinity to electrons has an ability of coordinating to a metal and on the excellent thermal resistance possessed by the pyridine ring. The present inventors have experimentally produced various organic EL devices using the compounds, and have extensively performed property evaluation of the devices. As a result, they have accomplished the invention.

Namely, the invention provides a compound having a triazine ring structure having pyridyl groups attached thereto, which is represented by the general formula (1); and an organic EL device comprising a pair of electrodes and at least one organic layer interposed between the electrodes, wherein the at least one of the organic layer(s) contains the compound:

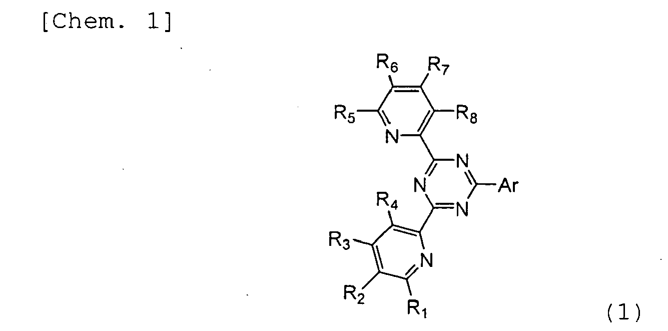

wherein Ar represents a substituted or unsubstituted aromatic hydrocarbon group, a substituted or unsubstituted aromatic heterocyclic group, or a substituted or unsubstituted condensed polycyclic aromatic group; R1 to R8 may be the same or different from one another and each independently represents a hydrogen atom, a fluorine atom, a trifluoromethyl group, a cyano group, an alkyl group, a substituted or unsubstituted aromatic hydrocarbon group, a substituted or unsubstituted aromatic heterocyclic group, or a substituted or unsubstituted condensed polycyclic aromatic group.

The aromatic hydrocarbon group, aromatic heterocyclic group or condensed polycyclic aromatic group in the substituted or unsubstituted aromatic hydrocarbon group, substituted or unsubstituted aromatic heterocyclic group, or substituted or unsubstituted condensed polycyclic aromatic group represented by Ar in the general formula (1) specifically includes the following groups: a phenyl group, a biphenylyl group, a terphenyl group, a tetrakisphenyl group, a styryl group, a naphthyl group, an anthryl group, an acenaphthenyl group, a fluorenyl group, a phenanthryl group, an indenyl group, a pyrenyl group, a pyridyl group, a pyrimidyl group, a furanyl group, a pyranyl group, a thiophenyl group, a quinolyl group, a benzofuranyl group, a benzothiophenyl group, an indolyl group, a carbazolyl group, a benzoxazolyl group, a quinoxalyl group, a benzimidazolyl group, a pyrazolyl group, a dibenzofuranyl group, a dibenzothiophenyl group, a naphthyridinyl group, a triazinyl group, and a benzothiazolyl group.

The substituent in the substituted aromatic hydrocarbon group, substituted aromatic heterocyclic group, or substituted condensed polycyclic aromatic group represented by Ar in the general formula (1) specifically includes groups such as a fluorine atom, a chlorine atom, a cyano group, a hydroxyl group, a nitro group, an alkyl group, a cycloalkyl group, an alkoxy group, an amino group, a phenyl group, a naphthyl group, an anthryl group, a fluorenyl group, a styryl group, a biphenylyl group, a pyridyl group, a pyridoindolyl group, a quinolyl group, a benzothiazolyl group, a benzothiophenyl group, and a triazinyl group. These substituents may be further substituted.

The aromatic hydrocarbon group, aromatic heterocyclic group, or condensed polycyclic aromatic group in the substituted or unsubstituted aromatic hydrocarbon group, substituted or unsubstituted aromatic heterocyclic group, or substituted or unsubstituted condensed polycyclic aromatic group, among the substituents of the pyridyl groups represented by R1 to R8 in the general formula (1), specifically includes a phenyl group, a biphenylyl group, a terphenyl group, a tetrakisphenyl group, a styryl group, a naphthyl group, an anthryl group, an acenaphthenyl group, a fluorenyl group, a phenanthryl group, an indenyl group, a pyrenyl group, a pyridyl group, a pyrimidyl group, a furanyl group, a pyranyl group, a thiophenyl group, a quinolyl group, a benzofuranyl group, a benzothiophenyl group, an indolyl group, a carbazolyl group, a benzoxazolyl group, a quinoxalyl group, a benzimidazolyl group, a pyrazolyl group, a dibenzofuranyl group, a dibenzothiophenyl group, and a naphthyridinyl group.
The substituent in the substituted aromatic hydrocarbon group, substituted aromatic heterocyclic group, or substituted condensed polycyclic aromatic group, among the substituents of the pyridyl groups represented by R1 to R8 in the general formula (1), specifically includes groups such as a fluorine atom, a chlorine atom, a cyano group, a hydroxyl group, a nitro group, an alkyl group having 1 to 6 carbon atoms, an alkoxy group, an amino group, a trifluoromethyl group, a naphthyl group, an aralkyl group, a fluorenyl group, an indenyl group, a pyridyl group, a pyrimidyl group, a furanyl group, a pyranyl group, a thiophenyl group, a quinolyl group, a benzofuranyl group, a benzothiophenyl group, an indolyl group, a carbazolyl group, a benzoxazolyl group, a quinoxalyl group, a benzimidazolyl group, a pyrazolyl group, a dibenzofuranyl group, and a dibenzothiophenyl group. These substituents may be further substituted.

The compound having a triazine ring structure having substituted or unsubstituted pyridyl groups attached thereto, which is represented by the general formula (1) of the invention, provides high electron mobility as compared with conventional electron-transporting materials, has an excellent hole-blocking ability, and is stable in a thin-film state.

The compound having a triazine ring structure having substituted or unsubstituted pyridyl groups attached thereto, which is represented by the general formula (1) of the invention, can be used as a constituent material for an electron-transporting layer of an organic EL device. The use of the compound of the invention exhibiting a higher electron injection/mobile rate as compared with conventional materials provides effects of improving electron transport efficiency from the electron-transporting layer to an emitting layer to enhance luminous efficiency and also lowering a driving voltage to enhance durability of the organic EL device.

The compound having a triazine ring structure having substituted or unsubstituted pyridyl groups attached thereto, which is represented by the general formula (1) of the invention, can be also used as a constituent material for a hole-blocking layer of an organic EL device. The use of the compound of the invention excellent in hole-blocking ability and also electron transport property as compared with conventional materials and having a high stability in a thin-film state provides effects of lowering a driving voltage, improving current resistance, and enhancing maximum emission luminance of the organic EL device, while exhibiting high luminous efficiency.

The compound having a triazine ring structure having substituted or unsubstituted pyridyl groups attached thereto, which is represented by the general formula (1) of the invention, can be also used as a constituent material for an emitting layer of an organic EL device. The use of an emitting layer prepared by using the material of the invention excellent in electron transport property as compared with conventional materials and having a wide band-gap as a host material for the emitting layer and making a fluorescent material or a phosphorescent material, called a dopant, carried thereon provides effects of realizing an organic EL device exhibiting a lowered driving voltage and having improved luminous efficiency.

Since the organic EL device of the invention uses the compound having a triazine ring structure having substituted or unsubstituted pyridyl groups and a substituted or unsubstituted aromatic hydrocarbon group, aromatic heterocyclic group, or condensed polycyclic aromatic group non-symmetrically attached thereto, which exhibits high electron mobility as compared with conventional electron-transporting materials, has an excellent hole-blocking ability, and is stable in a thin-film state, it becomes possible to realize a high efficiency and a high durability.

### ADVANTAGEOUS EFFECTS OF THE INVENTION

The compound having a triazine ring structure having substituted or unsubstituted pyridyl groups attached thereto according to the invention is useful as a constituent material for an electron-transporting layer, a hole-blocking layer, or an emitting layer of an organic EL device, and the luminous efficiency and durability of a conventional organic EL device can be improved by producing an organic EL device using the compound.

### BRIEF DESCRIPTION OF THE DRAWINGS

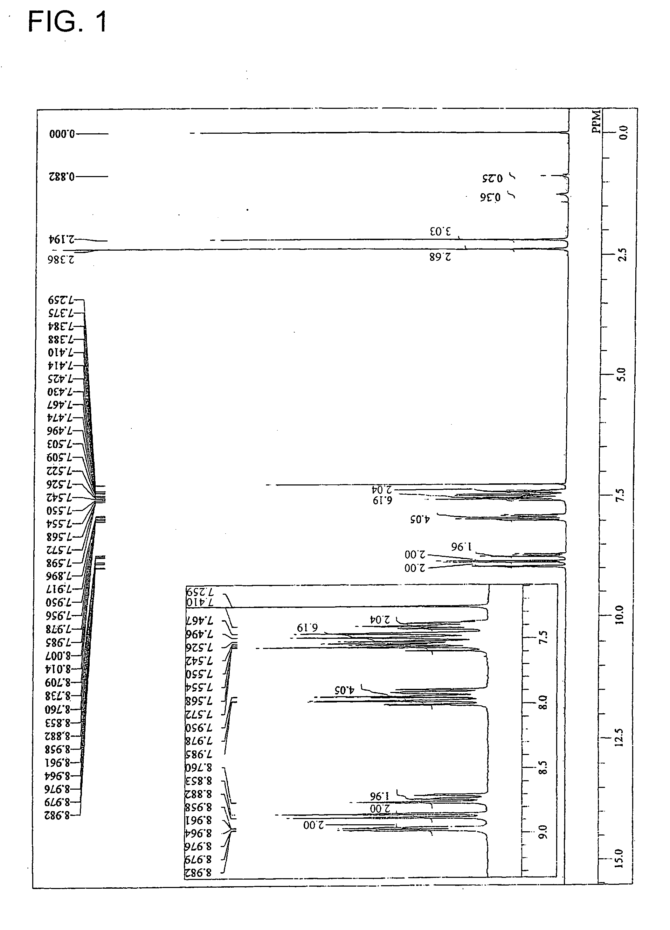

FIG. 1 is a 1H-NMR chart of Example 1.

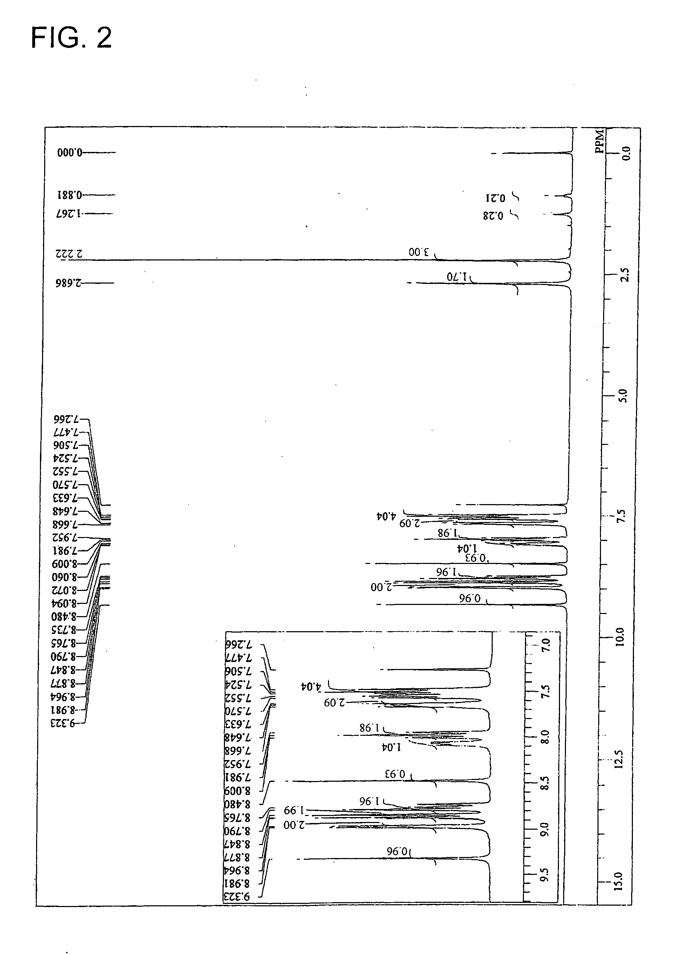

FIG. 2 is a 1H-NMR chart of Example 2.

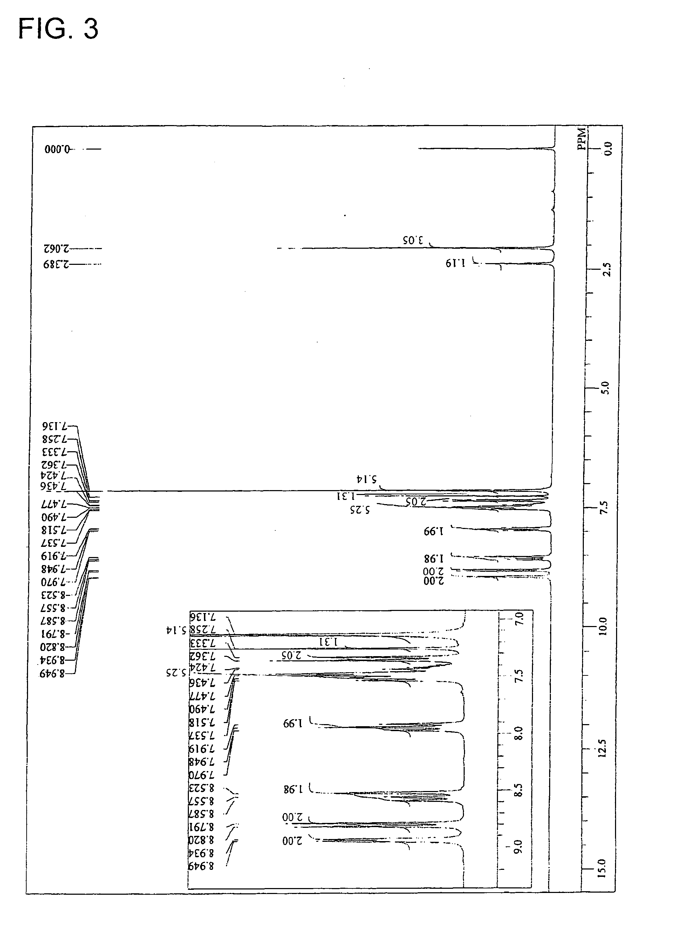

FIG. 3 is a 1H-NMR chart of Example 3.

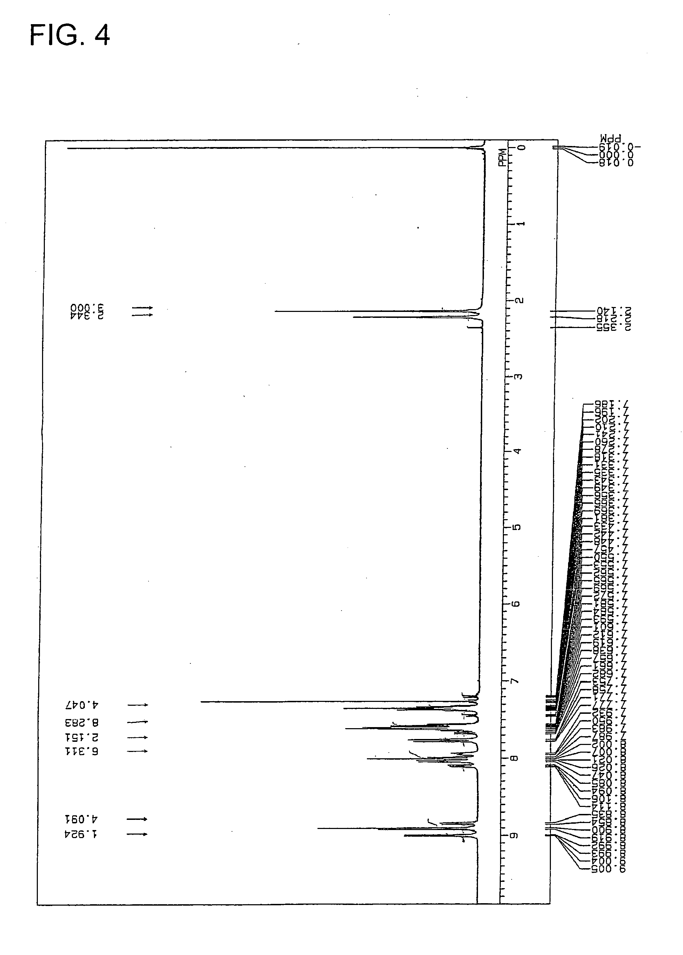

FIG. 4 is a 1H-NMR chart of Example 4.

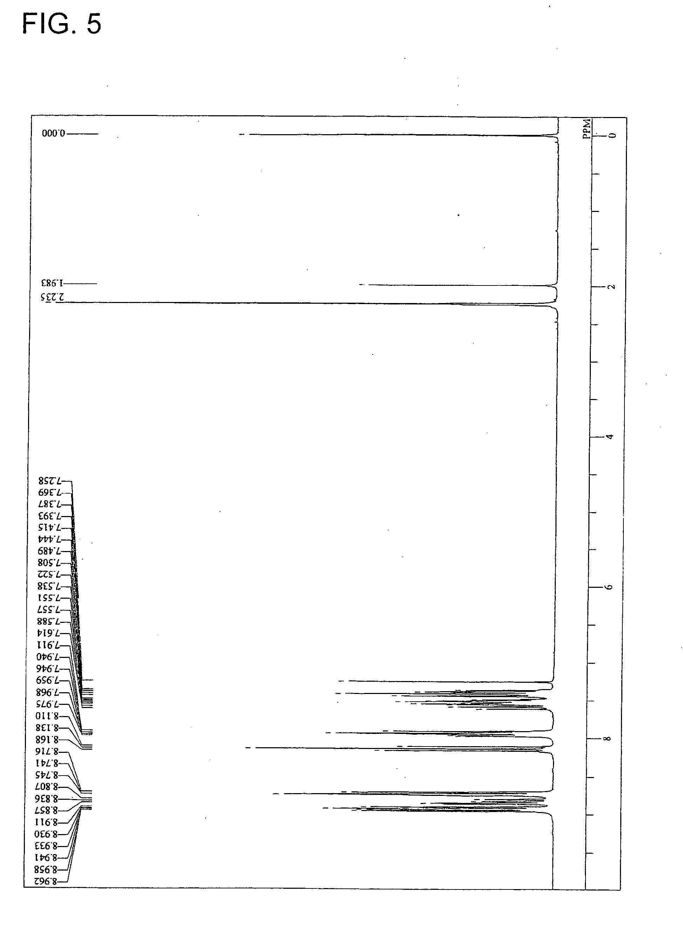

FIG. 5 is a 1H-NMR chart of Example 5.

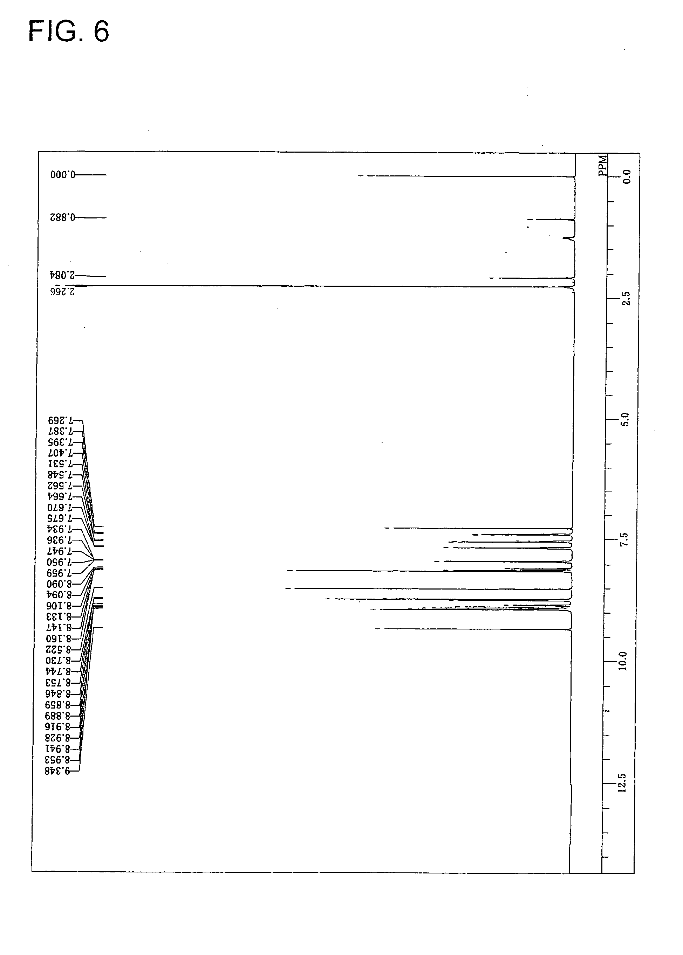

FIG. 6 is a 1H-NMR chart of Example 6.
FIG. 7 is a drawing showing the constitution of the EL devices of Examples 9 to 12.
FIG. 8 is a drawing showing the constitution of the EL device of Comparative Example 1.
FIG. 9 is a graph comparing voltage/current density properties of Example 9 and Comparative Example 1.
FIG. 10 is a graph comparing voltage/luminance properties of Example 9 and Comparative Example 1.
FIG. 11 is a graph comparing current density/luminance properties of Example 9 and Comparative Example 1.
FIG. 12 is a graph comparing current density/luminous efficiency of Example 9 and Comparative Example 1.

### DESCRIPTION OF REFERENCE NUMERALS AND SIGNS

- 1:: Glass substrate
- 2:: Transparent anode
- 3:: Hole-transporting layer
- 4:: Emitting layer
- 5:: Hole-blocking layer
- 6:: Electron-transporting layer
- 7:: Cathode

### BEST MODE FOR CARRYING OUT THE INVENTION

The compound having a triazine ring structure having substituted or unsubstituted pyridyl groups attached thereto according to the invention is a novel compound, and the compound can be synthesized, for example, by subjecting a lithium amidinide salt, which is formed of a cyano-substituted compound of a corresponding aromatic hydrocarbon compound, aromatic heterocyclic compound, or condensed polycyclic aromatic compound with a lithium alkylamide, and a substituted cyanopyridine to a cyclization reaction, to thereby synthesize a compound having a triazine ring structure having substituted or unsubstituted pyridyl groups attached thereto (see, e.g., Non-Patent Document 7).

Further, by subjecting a compound wherein one or more hydrogen atoms are replaced by halogen atom(s) in the aromatic hydrocarbon group, aromatic heterocyclic group, or condensed polycyclic aromatic group represented by Ar in the general formula (1) and an arylboronic acid to a cross-coupling reaction such as the Suzuki coupling (see, e.g., Non-Patent Document 8), a substituted or unsubstituted aromatic hydrocarbon group, a substituted or unsubstituted aromatic heterocyclic group, or a substituted or unsubstituted condensed polycyclic aromatic group can be further introduced.

Non-Patent Document 7: Matthew I. J. Polson et al., Chem. Eur. J. 10, 3640 (2004)
Non-Patent Document 8: N. Miyaura et al., Synth. Commun., 11, 513 (1981)

Among the compounds having a triazine ring structure having substituted or unsubstituted pyridyl groups attached thereto represented by the general formula (1), specific examples of preferred compounds are shown below, but the invention is not limited to these compounds.

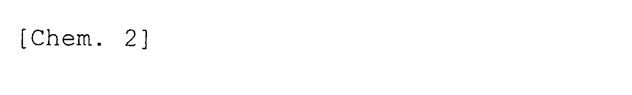

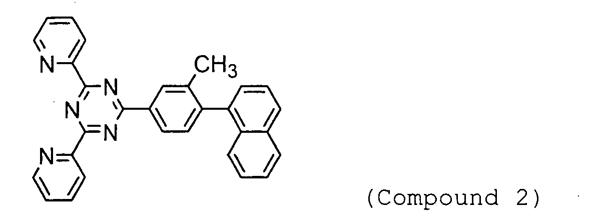

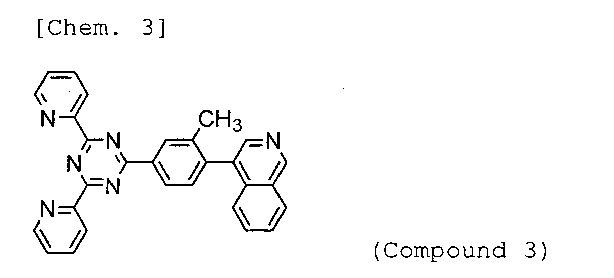

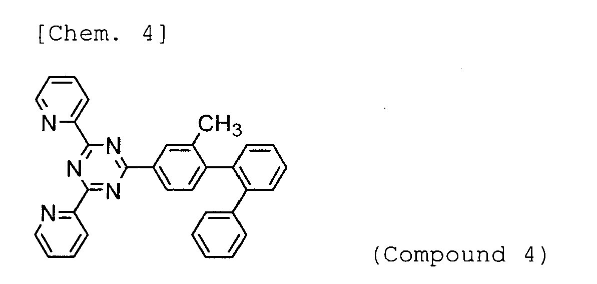

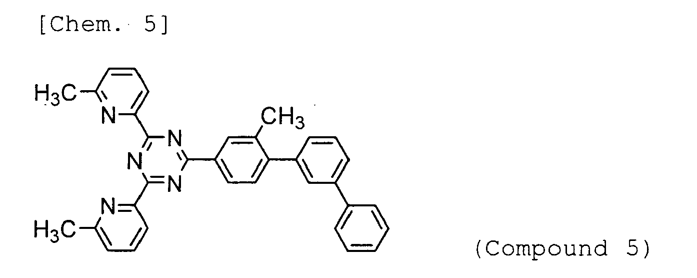

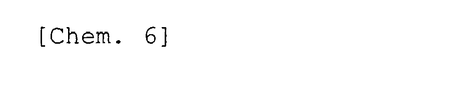

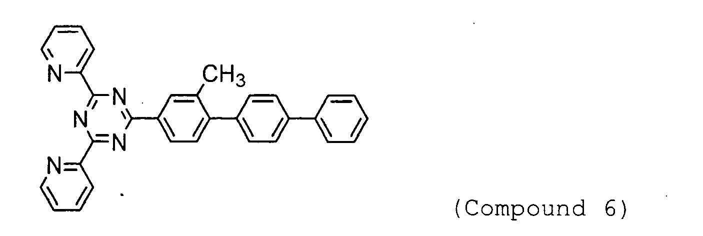

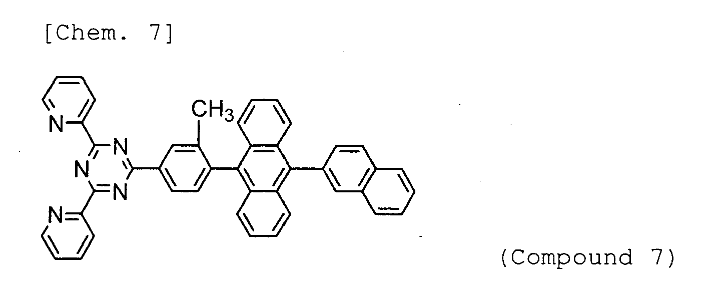

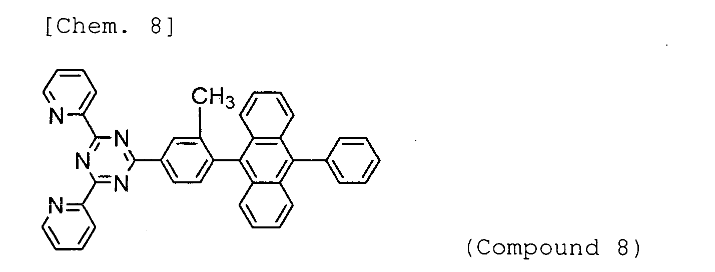

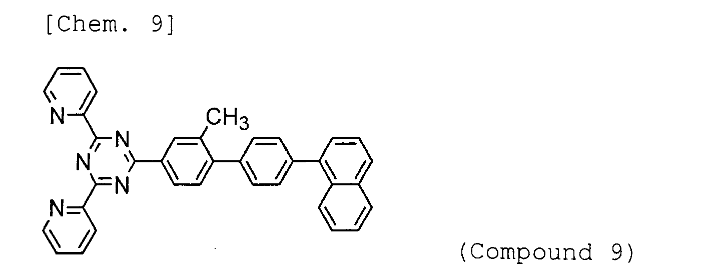

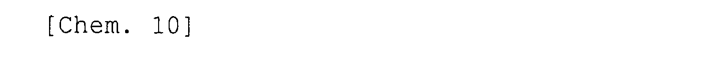

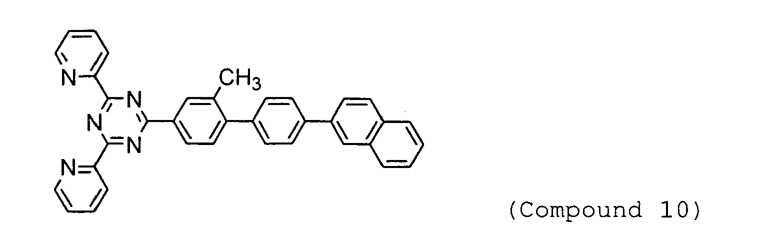

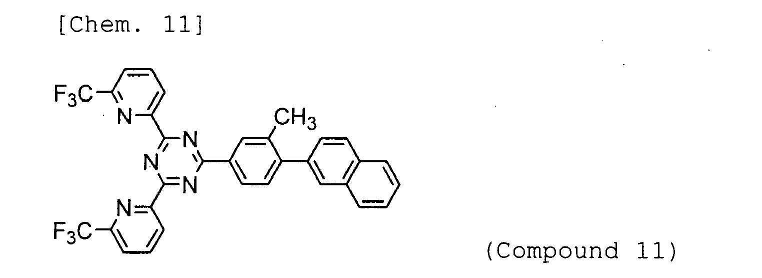

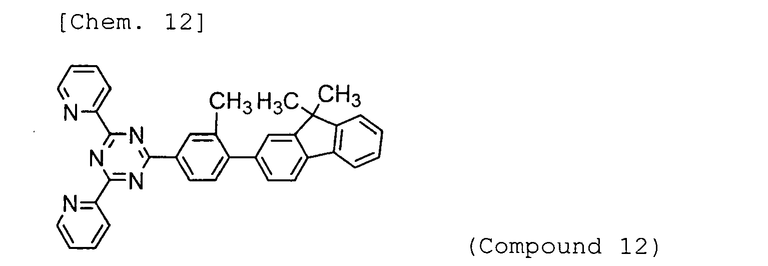

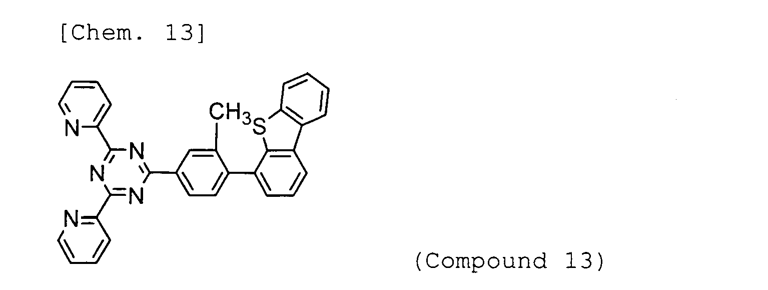

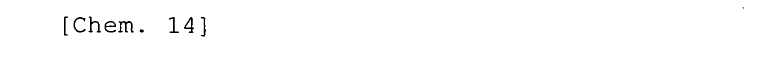

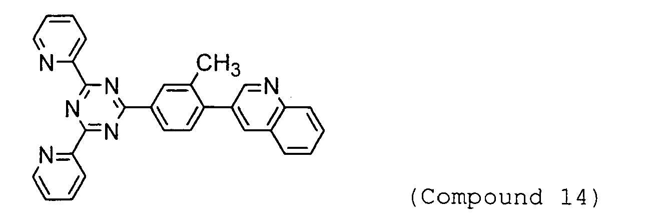

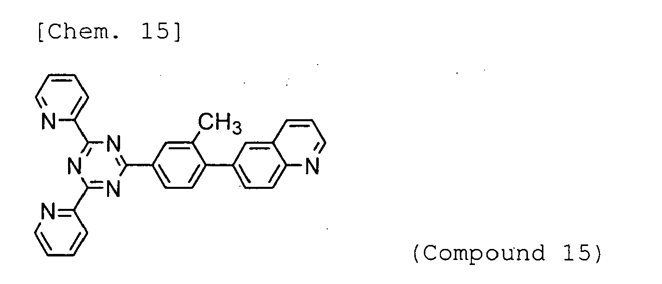

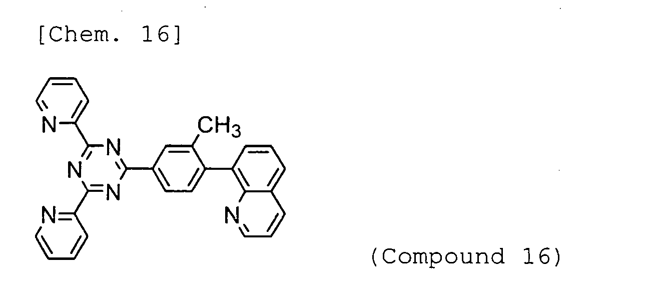

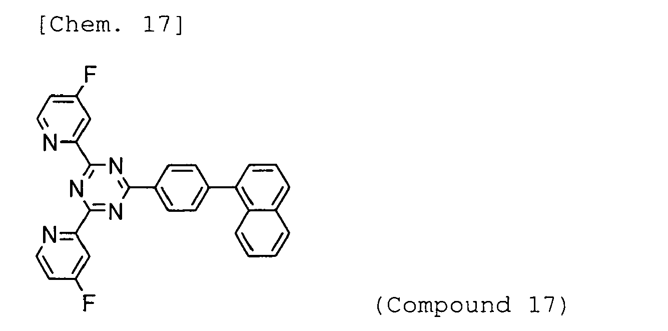

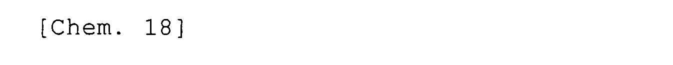

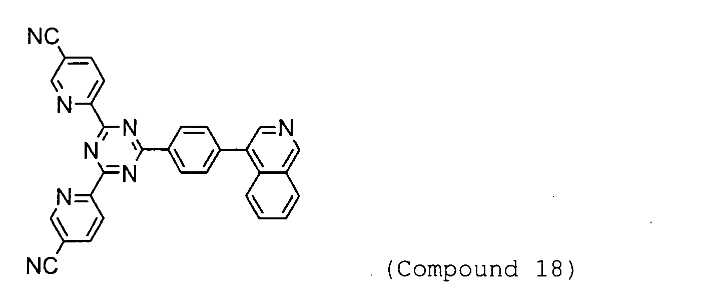

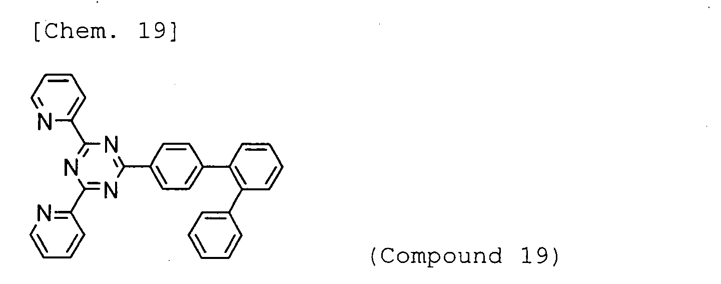

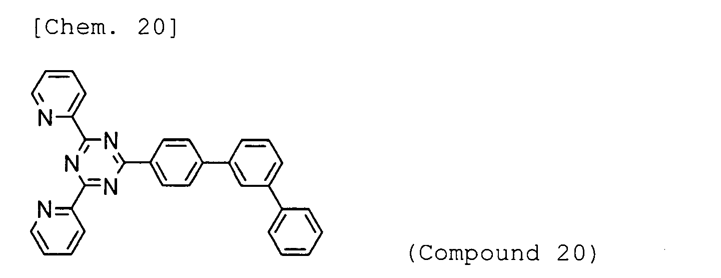

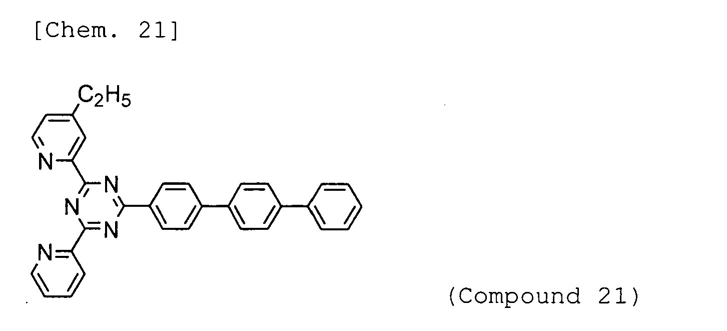

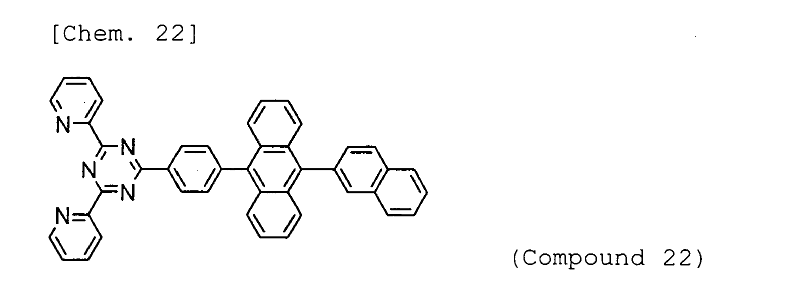

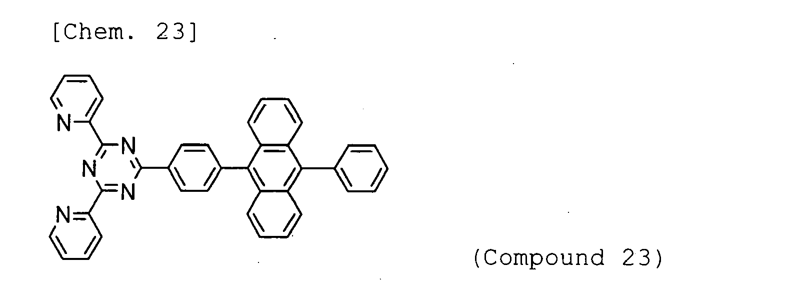

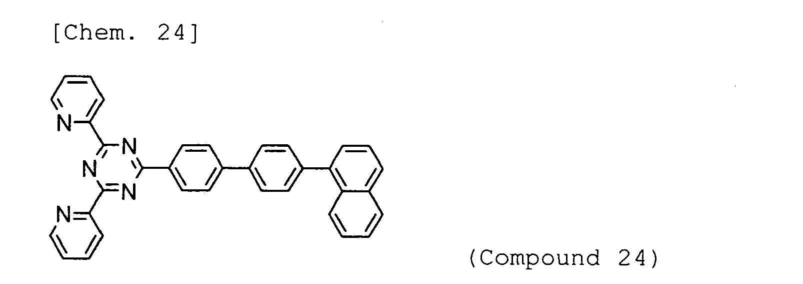

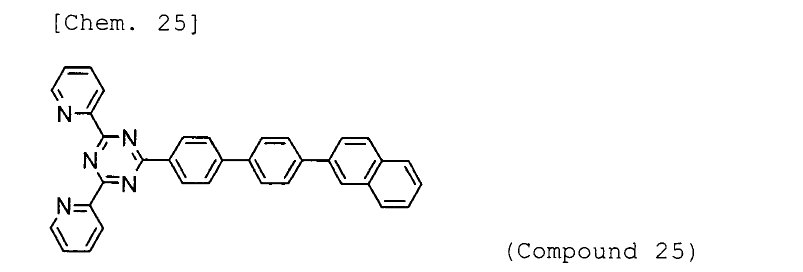

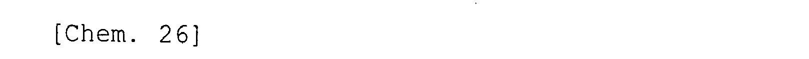

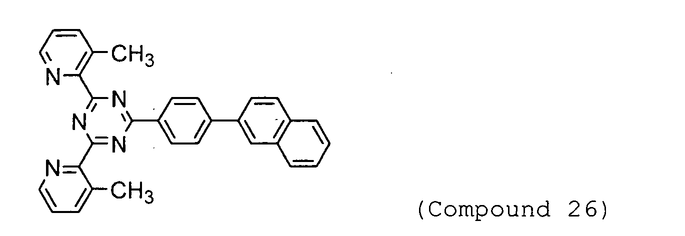

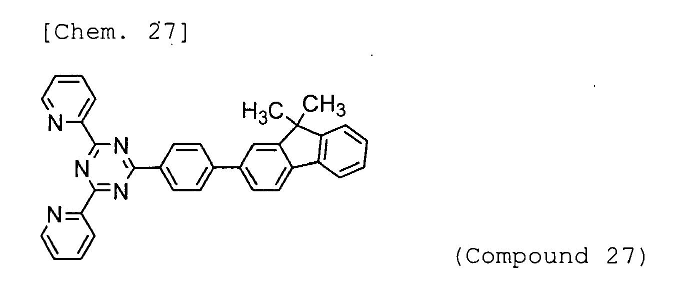

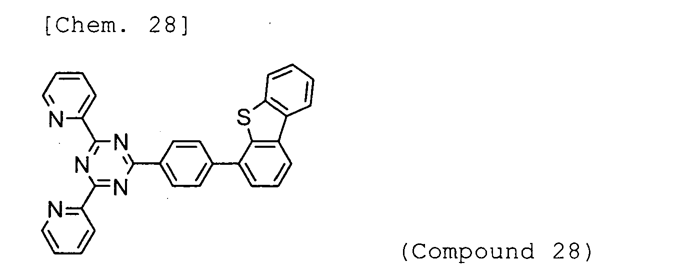

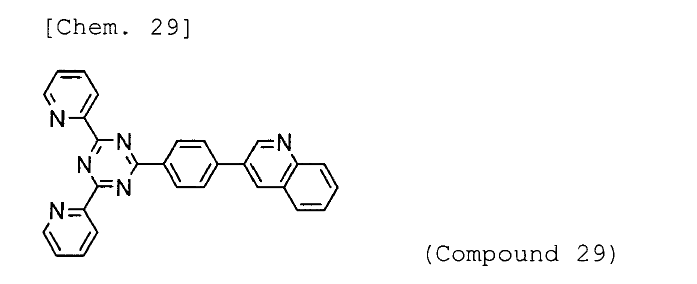

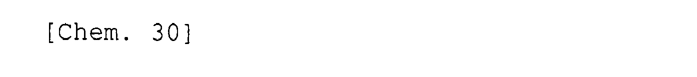

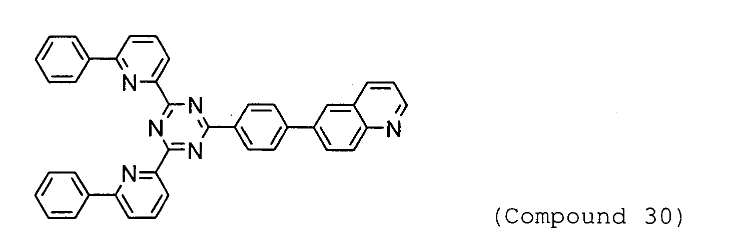

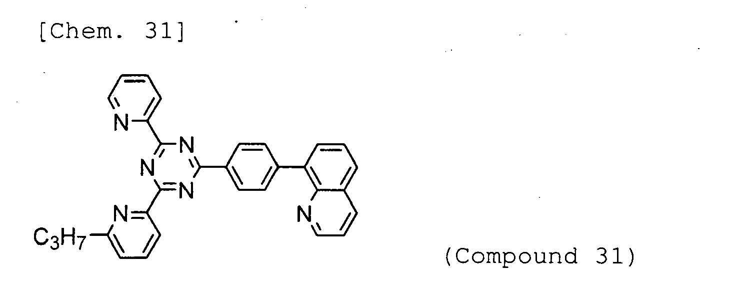

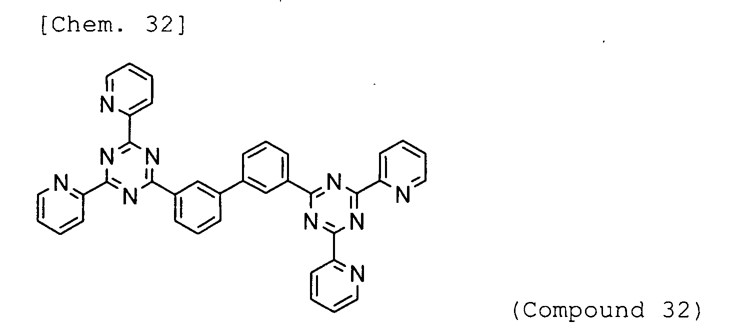

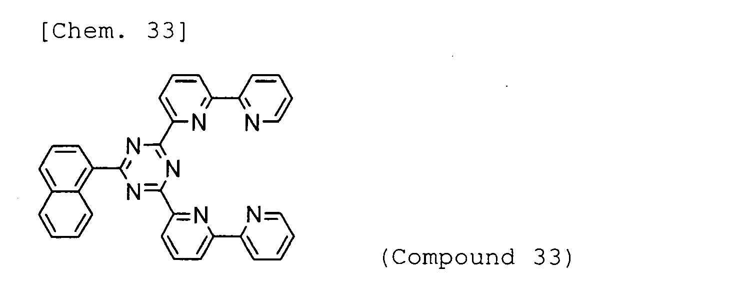

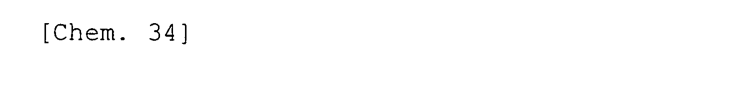

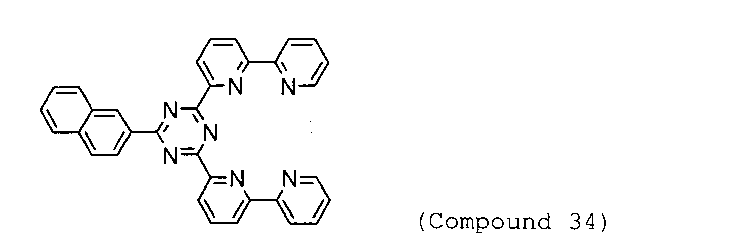

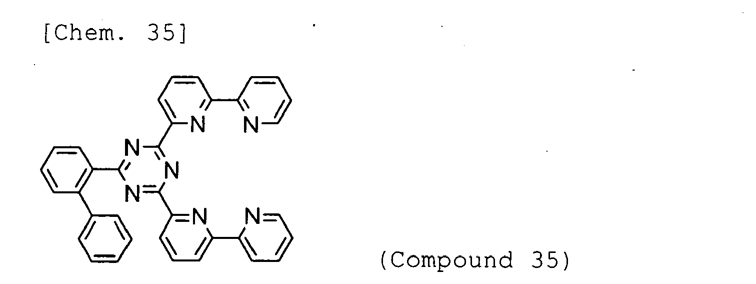

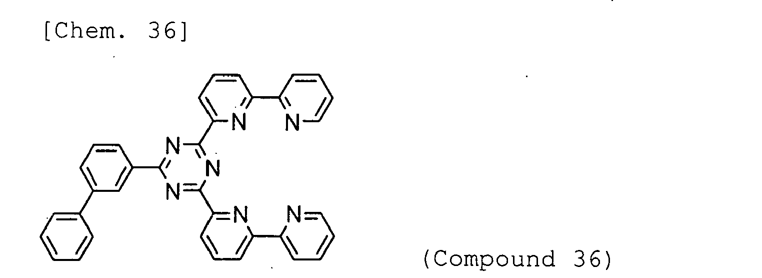

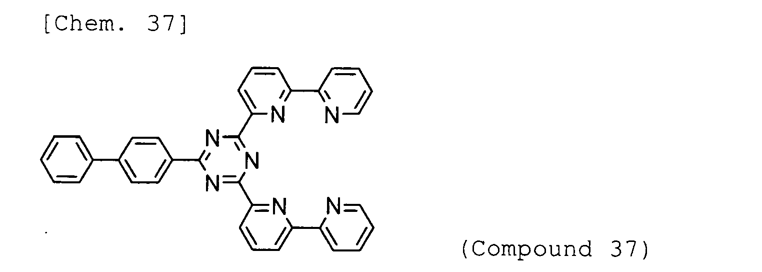

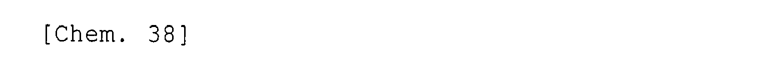

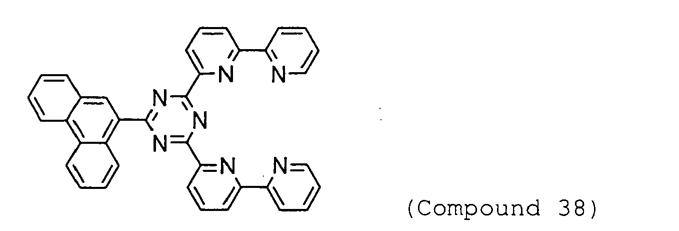

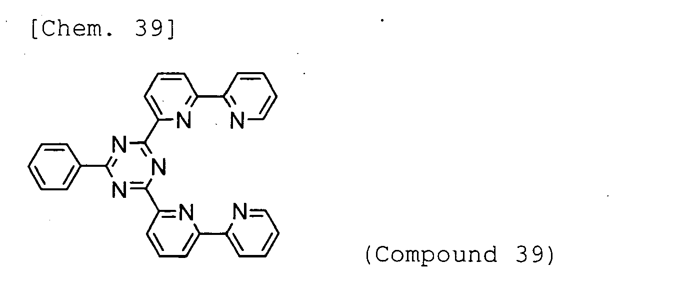

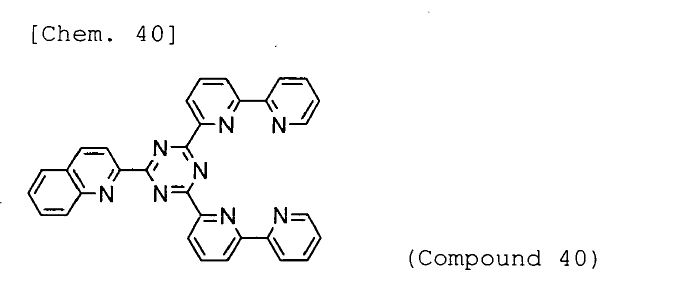

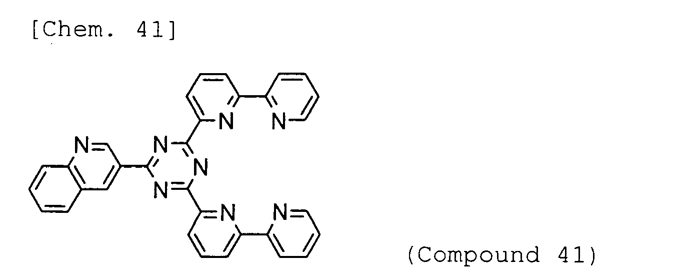

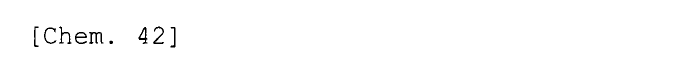

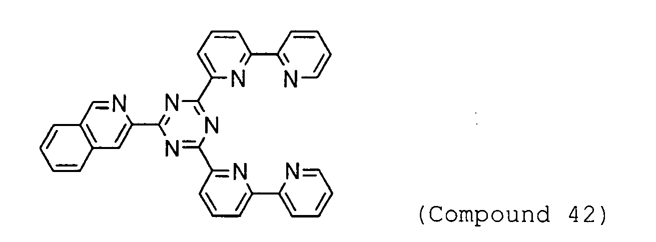

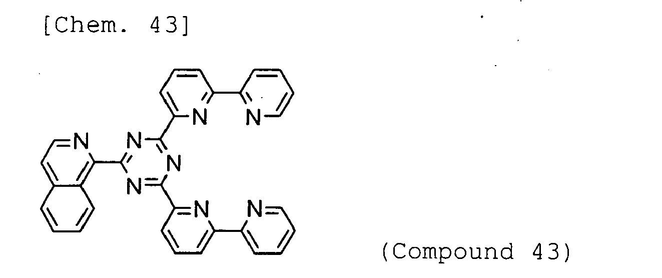

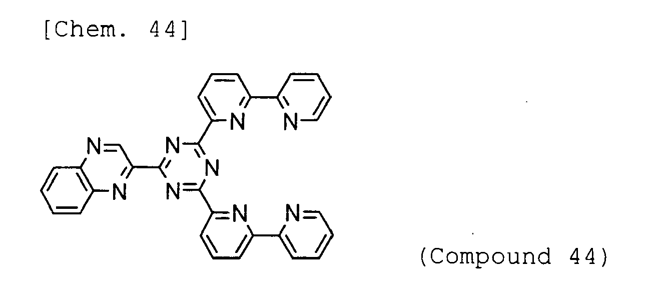

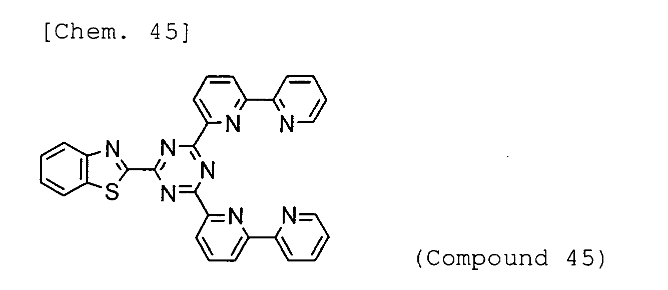

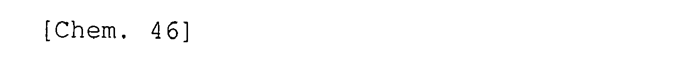

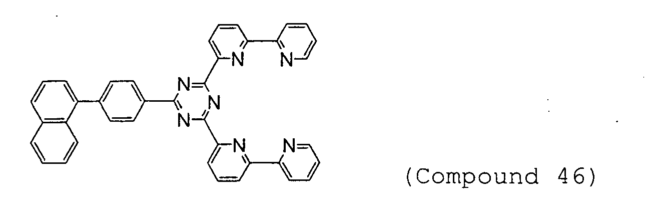

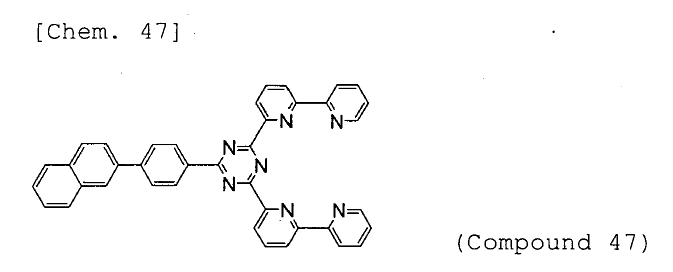

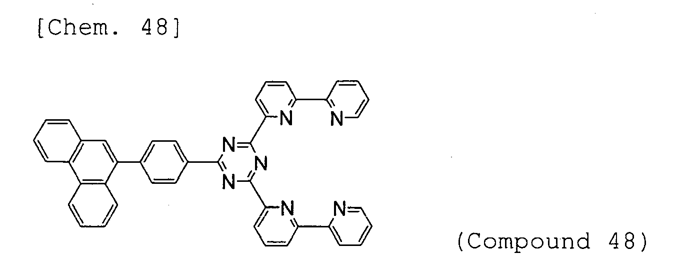

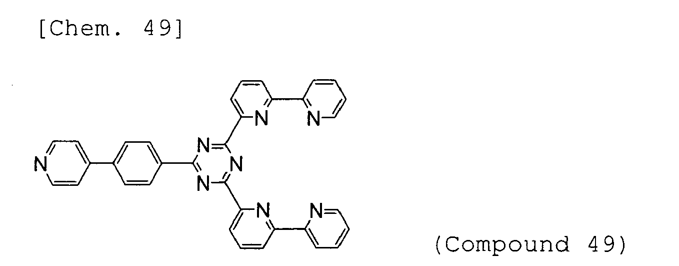

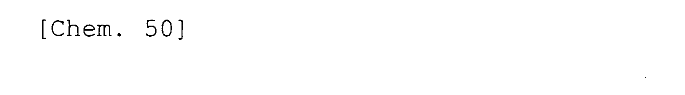

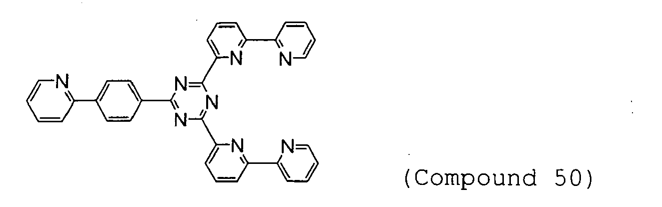

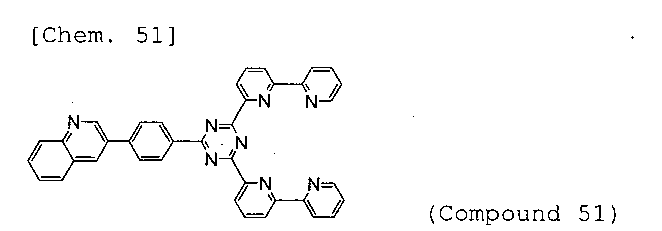

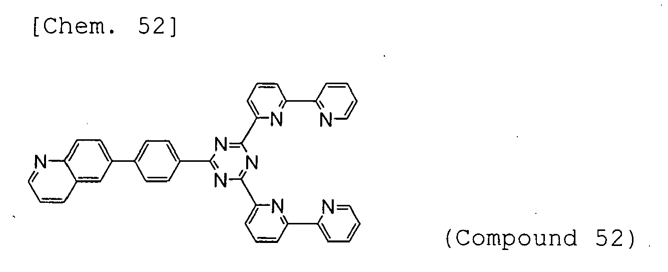

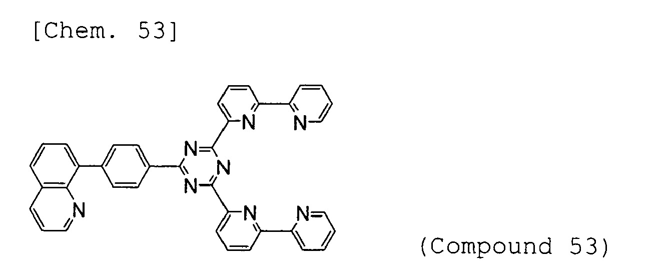

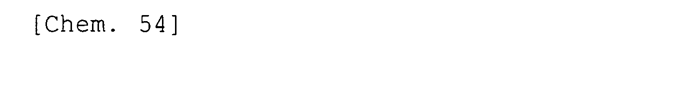

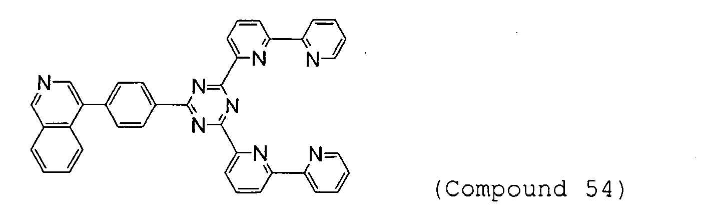

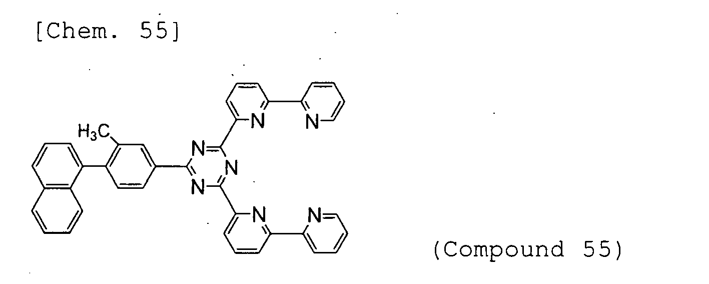

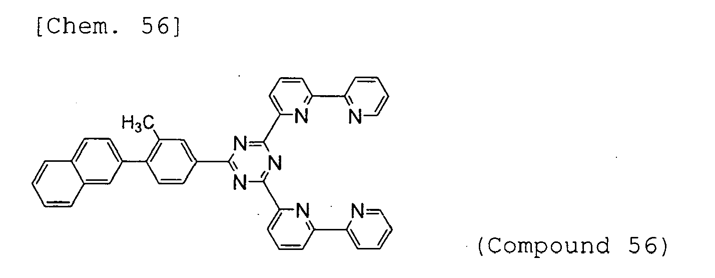

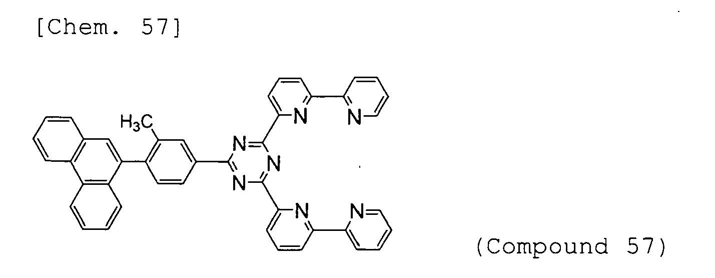

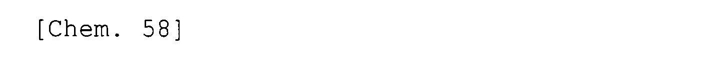

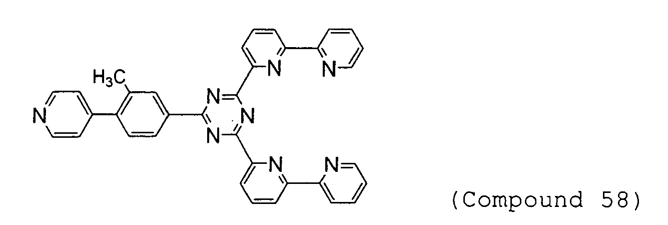

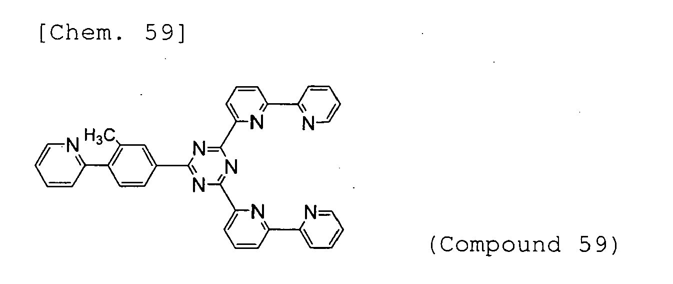

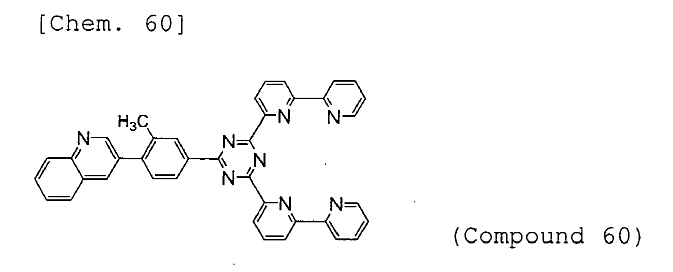

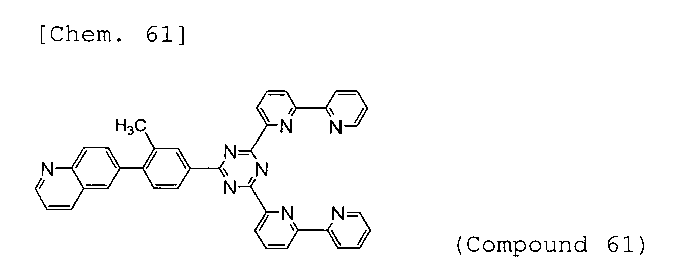

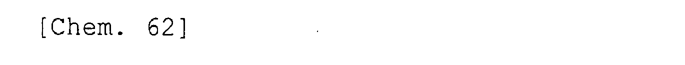

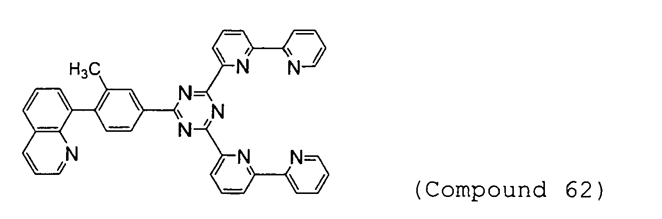

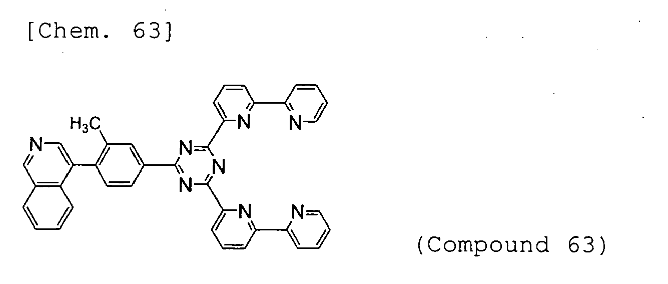

Embodiments of the present invention will be illustrated in greater detail with reference to the following Examples, but the invention should not be construed as being limited thereto so long as not exceeding the gist thereof.

### Example 1

(Synthesis of 2-[3-methyl-4-(naphthalen-1-yl)phenyl]-4,6-di-2-pyridinyl(1,3,5)triazine (hereinafter referred to as αNPy-TRZ) (Compound 2))
1.0 g of 2-(4-Bromo-3-methylphenyl)-4,6-di-2-pyridinyl(1,3,5)triazine, 0.63 g of 1-naphthaleneboronic acid, 7.2 ml of a 1M potassium carbonate aqueous solution, 0.14 g of tetrakis(triphenylphosphine)palladium(0), 24 ml of toluene, and 6 ml of ethanol were added and the whole was stirred for 5 hours under heating and refluxing. After cooling to room temperature, water was added thereto for washing, thereby obtaining a crude product. The resulting crude product was dissolved in chloroform and purified by column chromatography (carrier: NH silica gel, eluent: chloroform/hexane) to obtain 0.97 g (yield 88%) of white crystals of αNPy-TRZ (Compound 2). The results of NMR analysis (CDCl3) were as follows. 8.975 (2H), 8.956 (2H), 8.840-7.700 (2H), 7.973-7.890 (4H), 7.559-7.408 (8H), 2.191 (3H).

### Example 2

### (Synthesis of 2- [3-methyl-4- (isoquinoline-4-yl) phenyl]-4,6-di-2-pyridinyl(1,3,5)triazine (hereinafter referred to as iQPy-TRZ) (Compound 3))

1.0 g of 2-(4-Bromo-3-methylphenyl)-4,6-di-2-pyridinyl(1,3,5)triazine, 0.64 g of 4-isoquinolineboronic acid, 7.2 ml of a 1M potassium carbonate aqueous solution, 0.14 g of tetrakis(triphenylphosphine)palladium(0), 24 ml of toluene, and 6 ml of ethanol were added and the whole was stirred for 8 hours under heating and refluxing. After cooling to room temperature, water was added thereto for washing, thereby obtaining a crude product. The resulting crude product was dissolved in chloroform and purified by column chromatography (carrier: NH silica gel, eluent: chloroform/hexane) to obtain 0.79 g (yield 71%) of white crystals of iQPy-TRZ (Compound 3). The results of NMR analysis (CDCl3) were as follows. 9.323 (1H), 8.973 (2H), 8.862 (2H), 8.790-8.755 (2H), 8.480 (1H), 8.140-7.952 (3H), 7.668-7.477 (6H), 2.222 (3H).

### Example 3

### (Synthesis of 2-[3-methyl-4-(biphenyl-2-yl)phenyl]-4,6-di-2-pyridinyl(1,3,5)triazine (hereinafter referred to as BPPy-TRZ) (Compound 4))

1.0 g of 2-(4-Bromo-3-methylphenyl)-4,6-di-2-pyridinyl(1,3,5)triazine, 0.73 g of 2-biphenyl-2-ylboronic acid, 7.2 ml of a 1M potassium carbonate aqueous solution, 0.14 g of tetrakis(triphenylphosphine)-palladium(0), 24 ml of toluene, and 6 ml of ethanol were added and the whole was stirred for 8 hours under heating and refluxing. After cooling to room temperature, water was added thereto for washing, thereby obtaining a crude product. The resulting crude product was dissolved in chloroform and purified by column chromatography (carrier: NH silica gel, eluent: chloroform/hexane) to obtain 0.90 g (yield 76%) of white crystals of BPPy-TRZ (Compound 4). The results of NMR analysis (CDCl3) were as follows. 8.942 (2H), 8.820 (2H), 8.587-8.523 (2H), 7.970-7.919 (2H), 7.537-7.333 (7H), 7.136 (5H), 2.062 (3H).

### Example 4

### (Synthesis of 2-[3-methyl-4-[10-( naphthalene-2-yl)anthracen-9-yl]phenyl]-4,6-di-2-pyridinyl(1,3,5)triazine (hereinafter referred to as βNAPPy-TRZ) (Compound 7))

1.0 g of 2-(4-Bromo-3-methylphenyl)-4,6-di-2-pyridinyl(1,3,5)triazine, 1.28 g of 10-( naphthalene-2-yl)anthracene-9-boronic acid, 7.2 ml of a 1M potassium carbonate aqueous solution, 0.06 g of tetrakis(triphenylphosphine)palladium(0), 23 ml of toluene, and 8 ml of ethanol were added and the whole was stirred for 10 hours under heating and refluxing. After cooling to room temperature, water was added thereto for washing, thereby obtaining a crude product. The resulting crude product was dissolved in chloroform and purified by column chromatography (carrier: NH silica gel, eluent: chloroform/hexane) to obtain 1.22 g (yield 40%) of yellow crystals of βNAPPy-TRZ (Compound 7). The results of NMR analysis (CDCl3) were as follows. 9.005 (2H), 8.919-8.835 (4H), 8.114-7.932 (6H), 7.777-7.758 (2H), 7.753-7.550 (8H), 7.381-7.278 (4H), 2.140 (3H).

### Example 5

### (Synthesis of 2-[3-methyl-4- (naphthalene-1-yl) phenyl-4,6-bis(2,2'-bipyridin-6-yl)(1,3,5)triazine (hereinafter referred to as αNBPy-TRZ) (Compound 55))

3.0 g of 2-(4-Bromo-3-methylphenyl)-4,6-bis(2,2'-bipyridin-6-yl)(1,3,5)triazine, 1.87 g of 1-naphthaleneboronic acid, 21.9 ml of a 1M potassium carbonate aqueous solution, 0.42 g of tetrakis(triphenylphosphine)palladium(0), 68 ml of toluene, and 17 ml of ethanol were added and the whole was stirred for 5.5 hours under heating and refluxing. After cooling to room temperature, water was added thereto for washing, thereby obtaining a crude product. The resulting crude product was washed with methanol to obtain 3.03 g (yield 55%) of white crystals of αNBPy-TRZ (Compound 55). The results of NMR analysis (CDCl3) were as follows. 8.962-8.716 (10H), 8.168-8.110 (2H), 7.975-7.911 (4H), 7.614-7.369 (8H), 2.235 (3H).

### Example 6

### (Synthesis of 2-[3-methyl-4- (isoquinoline-4-yl) phenyl]-4,6-bis(2,2'-bipyridin-6-yl)(1,3,5)triazine (hereinafter referred to as iQBPy-TRZ) (Compound 63))

3.88 g of 2-(4-Bromo-3-methylphenyl)-4,6-bis(2,2'-bipyridin-6-yl)-2-pyridinyl(1,3,5)triazine, 3.64 g of 4-isoquinolineboronic acid, 28.5 ml of a 1M potassium carbonate aqueous solution, 0.55 g of tetrakis(triphenylphosphine)palladium(0), 84 ml of toluene, and 21 ml of ethanol were added and the whole was stirred for 5 hours under heating and refluxing. After cooling to room temperature, water was added thereto for washing, thereby obtaining a crude product. The resulting crude product was washed with methanol, dissolved in chloroform, and purified by column chromatography (carrier: NH silica gel, eluent: chloroform/hexane) to obtain 3.44 g (yield 88%) of white crystals of iQBPy-TRZ (Compound 63). The results of NMR analysis (CDCl3) were as follows. 9.348 (1H), 8.953-8.846 (6H), 8.753-8.730 (4H), 8.522 (1H), 8.160-8.090 (3H), 7.950-7.947 (2H), 7.670-7.664 (2H), 7.562-7.531 (2H), 7.407-7.387 (2H), 2.266 (3H).

### Example 7

For the compounds of the invention, melting point and glass transition point were determined by means of a highly sensitive differential scanning calorimeter (DSC 3100S manufactured by Bruker AXS).

| | Melting Point | Glass Transition Point |
|---|---|---|
| αNPy-TRZ (Compound 2) | 121.9°C | 96.1°C |
| iQPy-TRZ (Compound 3) | 227.2°C | 100.2°C |
| BPPy-TRZ (Compound 4) | 219.0°C | 90.4°C |
| βNAPPy-TRZ (Compound 7) | 347.5°C | 171.1°°C |
| αNBPy-TRZ (Compound 55) | 219.2°C | 104.0°C |
| iQBPy-TRZ (Compound 63) | 218.1°C | 108.0°C |

The compounds of the invention show a high glass transition point and thus are stable in a thin-film state.

### Example 8

Using each of the compounds of the invention, a deposited film having a film thickness of 100 nm was prepared on an ITO substrate and work function was measured on an atmospheric photoelectron spectrometer (AC3 type, manufactured by Riken Keiki Co., Ltd.).

| | Work Function |
|---|---|
| αNPy-TRZ (Compound 2) | 6.54 eV |
| iQPy-TRZ (Compound 3) | 6.32 eV |
| BPPy-TRZ (Compound 4) | 6.41 eV |

Thus, the compounds of the invention have values deeper than a work function of 5.4 eV possessed by common hole-transporting materials such as NPD and TPD and have a large hole-blocking ability.

### Example 9

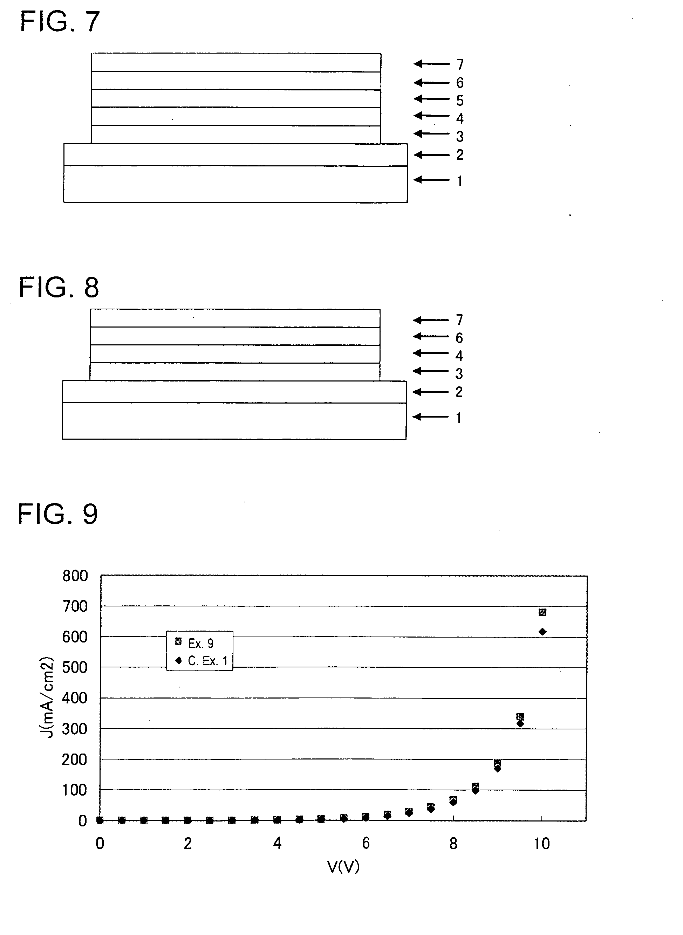

An organic EL device was prepared by depositing a hole-transporting layer 3, an emitting layer 4, a hole-blocking layer 5, an electron-transporting layer 6, and a cathode (magnesium electrode) 7 in this order on a glass substrate 1 on which an ITO electrode had been formed as a transparent anode 2 in advance, as shown in FIG. 7. After the glass substrate 1 on which ITO having a film thickness of 150 nm had been formed was washed with an organic solvent, the surface was washed by UV ozone treatment. It was mounted in a vacuum deposition machine, which was then evacuated to 0.001 Pa or lower.

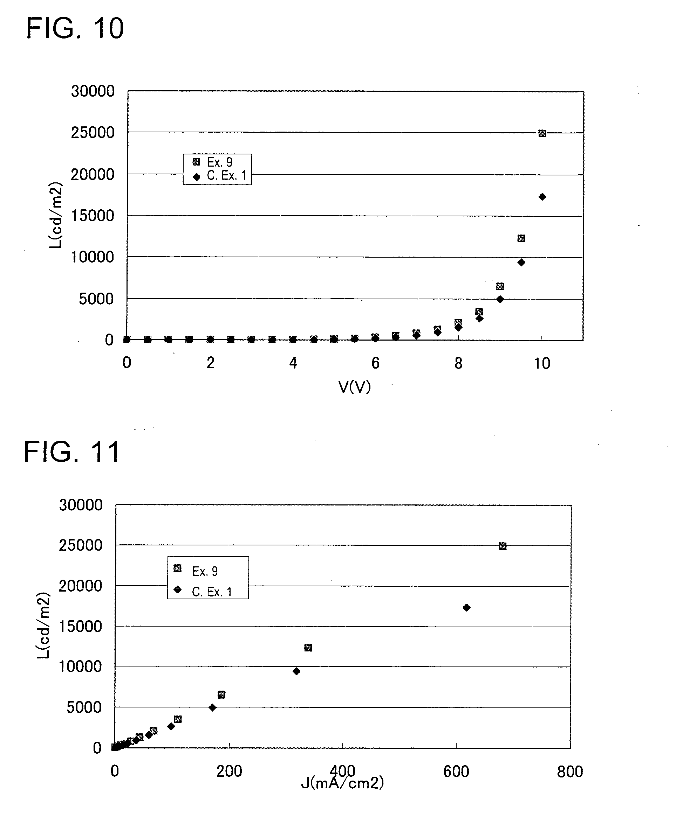

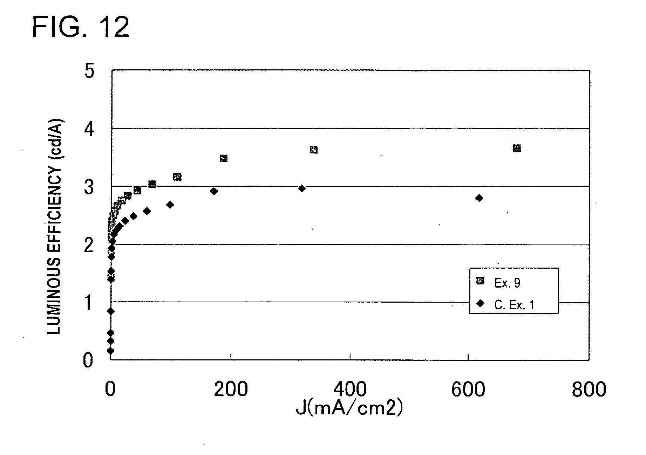

Subsequently, NPD was formed thereon at a deposition rate of 6 nm/min to a thickness of about 50 nm as the hole-transporting layer 3. As the emitting layer 4, Alq3 was formed thereon at a deposition rate of 6 nm/min to a thickness of about 20 nm. On the emitting layer 4, αNPy-TRZ (Compound 2) was formed at a deposition rate of 6 nm/min to a thickness of about 30 nm as the hole-blocking layer-cum-electron-transporting layer 5 and 6. Finally, the pressure was put back to atmospheric pressure and a mask for cathode deposition was inserted. Then, the pressure was again reduced and an alloy of MgAg was deposited in a ratio of 10:1 to a thickness of about 200 nm to form the cathode 7. The prepared device was stored in a vacuum desiccator and characteristic properties were measured in the atmosphere at ordinary temperature. The results were shown in FIG. 9 to FIG. 12.

As a result of applying direct voltage to the organic EL device of the invention thus formed, a luminescence of 100 cd/m² was observed from 5.07 V, and at 9.41 V, a current of 300 mA/cm² flowed to obtain a green luminescence of 10600 cd/m². The luminous efficiency at the luminance was 3.61 cd/A. Maximum luminance of the device before breakpoint was 24910 cd/m².

### Example 10

An organic EL device was prepared under the same conditions as in Example 9 except that the material of the hole-blocking layer-cum-electron-transporting layer 5 and 6 was replaced by iQPy-TRZ (Compound 3) of the invention. As a result of applying direct voltage to the device, a luminescence of 100 cd/m² was observed from 4.41 V, and at 8.79 V, a current of 300 mA/cm² flowed to obtain a green luminescence of 11200 cd/m². The luminous efficiency at the luminance was 3.68 cd/A. Maximum luminance of the device before breakpoint was 29500 cd/m².

### Example 11

An organic EL device was prepared under the same conditions as in Example 9 except that the material of the hole-blocking layer-cum-electron-transporting layer 5 and 6 was replaced by BPPy-TRZ (Compound 4) of the invention. As a result of applying direct voltage to the device, a luminescence of 100 cd/m² was observed from 4.63 V, and at 8.76 V, a current of 300 mA/cm² flowed to obtain a green luminescence of 10100 cd/m². The luminous efficiency at the luminance was 3.35 cd/A. Maximum luminance of the device before breakpoint was 20400 cd/m².

### Example 12

An organic EL device was prepared under the same conditions as in Example 9 except that the material of the hole-blocking layer-cum-electron-transporting layer 5 and 6 was replaced by iQBPy-TRZ (Compound 63) of the invention. As a result of applying direct voltage to the device, a luminescence of 100 cd/m² was observed from 4.35 V, and at 8.26 V, a current of 300 mA/cm² flowed to obtain a green luminescence of 9040 cd/m². The luminous efficiency at the luminance was 3.01 cd/A. Maximum luminance of the device before breakpoint was 26200 cd/m².

### [Comparative Example 1]

For comparison, an organic EL device was prepared under the same conditions as in Example 9 except that the material of the electron-transporting layer 6 was replaced by Alq3 and characteristic properties thereof were investigated. Namely, Alq3 was formed at a deposition rate of 6 nm/min to a thickness of about 50 nm as the emitting layer-cum-electron-transporting layer 4 and 6. A luminescence of 100 cd/m² was observed from 5.52 V, and at 9.46 V, a current of 300 mA/cm² flowed to obtain a green luminescence of 9400 cd/m². The luminous efficiency at the luminance was 2.96 cd/A. Maximum luminance of the device before breakpoint was 17350 cd/m².

Thus, it was revealed that the organic EL devices of the invention are excellent in luminous efficiency as compared with the devices using Alq3 that is employed as a common electron-transporting material.

While the invention has been described in detail and with reference to specific embodiments thereof, it will be apparent to one skilled in the art that various changes and modifications can be made therein without departing from the spirit and scope thereof.
The present application is based on Japanese Patent Application No. 2006-223725 filed on August 21, 2006, and the contents thereof are herein incorporated by reference.

### INDUSTRIAL APPLICABILITY

Since the compound having a triazine ring structure having substituted or unsubstituted pyridyl groups attached thereto according to the invention exhibits a good luminous efficiency and is stable in a thin-film state, the compound is excellent as a compound for organic EL devices. By preparing organic EL devices using the compound, device life and durability can be improved. For example, it becomes possible to spread the compound onto applications of electric home appliances and illumination.

## Claims

1. A compound having a triazine ring structure having pyridyl groups attached thereto, which is represented by the following general formula (1): 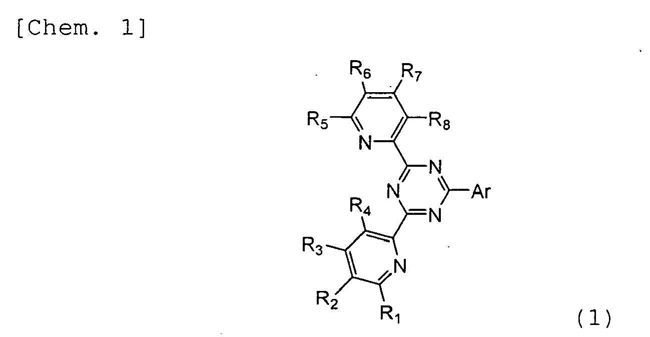 wherein Ar represents a substituted or unsubstituted aromatic hydrocarbon group, a substituted or unsubstituted aromatic heterocyclic group, or a substituted or unsubstituted condensed polycyclic aromatic group; R1 to R8 may be the same or different from one another and each independently represents a hydrogen atom, a fluorine atom, a trifluoromethyl group, a cyano group, an alkyl group, a substituted or unsubstituted aromatic hydrocarbon group, a substituted or unsubstituted aromatic heterocyclic group, or a substituted or unsubstituted condensed polycyclic aromatic group.

2. An organic electroluminescent device comprising a pair of electrodes and at least one organic layer interposed between the electrodes, wherein at least one of the organic layer(s) contains the compound having a triazine ring structure having pyridyl groups attached thereto according to claim 1.

3. The organic electroluminescent device according to claim 2, wherein the organic layer(s) comprises an electron-transporting layer, and the compound represented by the general formula (1) is present in the electron-transporting layer.

4. The organic electroluminescent device according to claim 2, wherein the organic layer(s) comprises a hole-blocking layer, and the compound represented by the general formula (1) is present in the hole-blocking layer.

5. The organic electroluminescent device according to claim 2, wherein the organic layer(s) comprises an emitting layer, and the compound represented by the general formula (1) is present in the emitting layer.

6. The organic electroluminescent device according to claim 2, wherein the organic layer(s) comprises an electron-injecting layer, and the compound represented by the general formula (1) is present in the electron-injecting layer.
